# EUROPÄISCHE PATENTSCHRIFT

(11) **EP 2 760 888 B1**
(45) Veröffentlichungstag und Bekanntmachung des Hinweises auf die Patenterteilung: **01.02.2017**
(21) Anmeldenummer: 12774983.6
(22) Anmeldetag: 26.09.2012
(51) Int. Cl.: C07K 16/16, C07K 16/40, A61K 39/395, A61P 1/04, A61P 37/04, C07K 16/02

(54) **IgY-ZUSAMMENSETZUNG FÜR DIE VERWENDUNG IN DER ZÖLIAKIE**
IgY COMPOSITION FOR USE IN COELIAC DISEASE
COMPOSITION D'IgY DESTINÉE À ÊTRE UTILISÉE CONTRE LA MALADIE CELIAQUE

(30) Priorität: 26.09.2011 DE 102011118028; 06.10.2011 DE 102011118015
(43) Veröffentlichungstag der Anmeldung: 06.08.2014
(73) Patentinhaber: Charité - Universitätsmedizin Berlin, 10117 Berlin (DE)
(72) Erfinder: SKRINER, Karl, 10557 Berlin (DE)
(74) Vertreter: Gulde, Klaus W.
(86) Internationale Anmeldenummer: PCT/EP2012/068914
(87) Internationale Veröffentlichungsnummer: WO 2013/045469

(56) Entgegenhaltungen:
- WO-A1-2011/039350
- WO-A2-2010/116196
- US-A1- 2011 008 362

## Beschreibung

Die Zöliakie (nach ICD-10, Version WHO 2006: K90.0), auch Gluten-sensitive oder Gluteninduzierte Enteropathie, intestinaler Infantilismus; bei Erwachsenen auch nichttropische oder einheimische Sprue, Gluten-Unverträglichkeit oder Heubner- Herter-Krankheit genannt, ist eine chronische Erkrankung der Dünndarmschleimhaut aufgrund einer Überempfindlichkeit gegen Gluten, ein Protein das in vielen Getreidesorten vorkommt. Die Unverträglichkeit bleibt lebenslang bestehen, sie ist zum Teil genetisch determiniert und kann derzeit nicht ursächlich behandelt werden.

Durch Gluten-haltige oder transglutminierte Nahrungsmittel entsteht eine Entzündung der Dünndarmschleimhaut mit oft ausgedehnter Zerstörung der Darmepithelzellen. Dadurch können Nährstoffe nur schlecht aufgenommen werden und verbleiben unverdaut im Darm. Symptome sind dementsprechend Gewichtsverlust, Durchfall, Erbrechen, Appetitlosigkeit, Müdigkeit, Misslaunigkeit und im Kindesalter nicht zuletzt eine Gedeihstörung. Die Schwere des Krankheitsbildes kann sehr unterschiedlich sein, was eine frühzeitige Diagnose erschwert. Eine nicht therapierte Zöliakie erhöht die Gefahr des Auftreten eines Non-Hodgkin-Lymphoms sowie auch für Karzinome des Verdauungstrakts, wie Darmkrebs.

Inzwischen sind eine Reihe von schädigenden Peptidfragmenten des Glutens identifiziert worden. Sie gehören alle der alkohollöslichen Fraktion an (sogenannte Prolamine) und werden Gliadine genannt. Bei entsprechend veranlagten Menschen führen diese Peptidfragmente zu einer komplexen Reaktion der Darmschleimhaut und des Immunsystems. Schleimhautzellen des Dünndarmes produzieren vermehrt verschiedene HLA-Klassen (HLA I, DR und DQ). Bestimmte Gliadinpeptide binden an die vermehrt gebildeten HLA-DQ2. Diese Bindung wird dadurch verstärkt, dass aus der zahlreich im Peptid vorhandenen Aminosäure Glutamin Glutaminsäure gebildet wird. Diese Glutaminsäurebildung wird durch das Enzym Gewebstransglutaminase (tTG), insbesondere durch Gewebstransglutaminase 2 (tTG2) vermittelt. Es entsteht durch tTG2 modifiziertes Gluten bzw. Gliadin, welches kausal mit der Entwicklung einer Zöliakie und dem Fortschreiten der Erkrankung assoziiert ist. Mit dieser Veränderung passt der entsprechende Abschnitt des Gliadin besser in die "Taschen" der HLA-Proteine. Der Komplex aus Gliadinpeptid und HLA-DQ2 bindet wiederum an CD4+-T-Helferzellen und ruft in diesen eine vermehrte Produktion von verschiedenen entzündungsauslösenden Botenstoffen hervor, beispielsweise Interferon-γ, TNF-α, Interleukin-6 und Interleukin-2. Im weiteren Prozess der Entzündung werden verschiedene Antikörper gebildet. Neben Antikörpern gegen Gliadinpeptide selbst (Gliadin-Antikörper, AGA) treten auch sogenannte Autoantikörper gegen körpereigene Antigene auf. Die Gewebstransglutaminase, insbesondere tTG2, wurde als hauptsächlich verantwortliches Autoantigen identifiziert.

Aufgrund dieser Befunde wird die Zöliakie aus pathophysiologischer Sicht als eine Mischform aus Allergie und Autoimmunerkrankung verstanden. Dabei stellt die allergische Komponente in Form der Überempfindlichkeit gegen das körperfremde Protein Gliadin den auslösenden Faktor dar, während für die Ausprägung der Symptome die autoimmunologische Reaktion gegen körpereigene Strukturen verantwortlich ist. Letztlich endet der Entzündungsvorgang in einer Apoptose der Enterozyten, die schließlich zu einem mehr oder weniger ausgeprägten Verlust von Dünndarmzotten führt. Die so geschädigte Dünndarmschleimhaut ist nun nicht mehr in der Lage, die zugeführte Nahrung in ausreichendem Umfang in die Blutbahn zu überführen, weil die Resorptionsfläche verkleinert ist.

Die Behandlung der Zöliakie besteht derzeit ausschließlich in einer Gluten-freien Diät, also in der Vermeidung der Aufnahme von Gluten durch den Patienten. Eine Therapieform, die dem Patienten die Aufnahme von Gluten-haltigen oder transglutaminierten Nahrungsmitteln erlaubt oder, z.B. bei unbeabsichtigter Aufnahme von Gluten-haltigen oder trnasglutaminierten Nahrungsmitteln, die Folgen einer solchen Aufnahme für den Patienten mindert ist bislang nicht bekannt.

Aus US 2011/008362 ist die Herstellung einer IgY-Zusammensetzung bekannt, welche durch Immunisierung von Hennen mit Gliadin und Aufbereitung der Zusammensetzung aus dem Eigelb der gelegten Eier gewonnen wird.

Aufgabe der vorliegenden Erfindung ist es einen oder mehrere Nachteile des Standes der Technik zu vermindern oder zu vermeiden. Insbesondere ist es eine Aufgabe der vorliegenden Erfindung neue Mittel für die Behandlung von Zöliakie bereitzustellen.

Die Aufgabe wird gelöst durch Bereitstellung einer IgY-Zusammensetzung gemäß einem der Ansprüche 1 bis 4.

Die hierin offenbarte IgY-Zusamensetzung zeichnet sich dadurch aus, dass die IgY-Zusammensetzung IgY-Antikörper, Fragmente und/oder Fab-Fragmente davon enthält, die spezifisch an ein Peptid mit einer Aminosäuresequenz mit der SEQ ID No. 1 binden. Die IgY-Zusammensetzung kann dabei beispielsweise enthalten:
- IgY-Antikörper, die spezifisch an ein Peptid mit einer Aminosäuresequenz mit der SEQ ID No. 1 binden;
- Fragmente solcher IgY-Antikörper, wobei die Fragmente spezifisch an ein Peptid mit einer Aminosäuresequenz mit der SEQ ID No. 1 binden; und/oder
- Fab-Fragmente solcher IgY-Antikörpern, wobei die Fab-Fragmente spezifisch an ein Peptid mit einer Aminosäuresequenz mit der SEQ ID No. 1 binden.

Aus WO 2011/039350 A1 und DE 10 2009 045 268.0 sind Peptide bekannt, die eine Aminosäuresequenz mit der SEQ ID No. 1 enthalten oder daraus bestehen, sowie Antikörper, die an solche Peptide binden. In WO 2011/039350 A1 und DE 10 2009 045 268.0 wird die Verwendung solcher Peptide und Antikörper in der Diagnose von Zöliakie offenbart. Weder in WO 2011/039350 A1 noch in DE 10 2009 045 268.0 werden Antikörper vom Typ IgY offenbart, die spezifisch an ein Peptid mit der Aminosäuresequenz mit der SEQ ID No. 1 binden, geschweige denn deren Eignung für die Verwendung in der Therapie von Zöliakie.

Der vorliegenden Erfindung liegt die überraschende Erkenntnis zugrunde, dass die Immunisierung eierlegenden Geflügels mit einem Peptid enthaltend die Aminosäuresequenz mit der SEQ ID No. 1 zur Bildung von IgY-Antikörpern führt, die spezifisch an durch tTG2 modifizierte Gliadinpeptide binden, also an besonders pathologische Gliadinpeptide, bei denen mittels tTG2 aus im Gliadinpeptid vorhandener Aminosäure Glutamin Glutaminsäure gebildet wurde. Durch die spezifische Bindung der IgY-Antikörper an das modifizierte Gliadin passt der entsprechende Abschnitt des modifizierten Gliadinpeptids nicht mehr in die entsprechenden Bindungsstellen der HLA-Proteine. Es kann folglich kein Komplex aus modifiziertem Gliadinpeptid und HLA-DQ2 gebildet werden und die pathologische Signalkette ist unterbrochen. Die Ausbildung einer Zöliakie-Symptomatik kann somit unterbunden oder zumindest vermindert werden.

Es wurde gefunden, dass sich die erfindungsgemäße IgY-Zusammensetzung enthaltend IgY-Antikörper, Fragmente und/oder Fab-Fragmente davon, die spezifisch an ein Peptid mit einer Aminosäuresequenz mit der SEQ ID No. 1 binden, für eine Verwendung in der Therapie der Zöliakie eignet. Hat ein Zöliakie-Patient Gluten-haltige oder transglutaminierte Nahrungsmittel zu sich genommen oder ist eine solche Aufnahme zu erwarten, so kann durch Zufuhr der erfindungsgemäßen IgY-Zusammensetzung verhindert werden, dass der Patient entsprechende Zöliakie-Symptome entwickelt und/oder die Ausprägung der zu erwartenden Zöliakie-Symptomatik kann signifikant vermindert werden. Somit ist es Zöliakie-Patienten erstmals möglich die Ausbildung einer Zöliakie-Symptomatik infolge einer Aufnahme Gluten-haltiger oder transglutaminierter Nahrungsmittel medikamentös zu verhindern oder abzumildern. Dem Zöliakie-Patienten wird damit die Möglichkeit gegeben wenigstens in Ausnahmefällen, wie z.B. Familienfeiern o.ä., Gluten-haltige oder transglutaminierte Nahrungsmittel zu konsumieren und damit am normalen gesellschaftlichen Leben teilzunehmen.

Die vorliegende Erfindung bezieht sich auf eine in den Ansprüchen 1 bis 4 definierte IgY-Zusammensetzung. Dabei wird für die Zwecke der Erfindung unter einer IgY-Zusammensetzung jede Zusammensetzung verstanden, die IgY-Antikörper, Fragmente und/oder Fab-Fragmente davon enthält. Daneben kann die IgY-Zusammensetzung weitere Bestandteile aufweisen.

Die beanspruchte IgY-Zusammensetzung enthält IgY-Antikörper, Fragmente und/oder Fab-Fragmente davon. IgY-Antikörper sind eine Antikörperklasse, die in Geflügel vorkommt, insbesondere in Hühnern, Enten und Gänsen, und dort das funktionale Äquivalente zu IgG-Antikörpern in Säugetieren darstellen. IgY-Antikörper binden jedoch im Gegensatz zu IgG nicht an Protein A beziehungsweise Protein G und auch nicht an zelluläre Fc-Rezeptoren. Darüber hinaus aktivieren IgY-Antikörper nicht das Komplementsystem. IgY-Antikörper eignen sich besonders gut zur Verabreichung an Säugetiere (z.B. in Form eines Nahrungsergänzungsstoffes oder Medikamentes), da die IgY-Antikörper im Säuger in der Regel nicht antigen wirken und somit auch nach mehrfacher Verabreichung im Zielorganismus keine problematische Immunantwort auslösen.

Unter IgY-Antikörpern werden intakte, unfragmentierte Antikörper der IgY-Klasse verstanden mit einer konstanten Fc-Region und zwei variablen Fab-Regionen. In den variablen Fab-Regionen befinden sich die Antigenbindungsstellen (CDR = complementarity determining region), die dem IgY-Antikörper seine Bindungsspezifität verleihen. Aufbau und Gewinnung von IgY-Antikörpern sind dem Fachmann bekannt und z.B. ausführlich beschrieben in Rüdiger Schade, Irene Behn, Michael Erhard: Chicken Egg Yolk Antibodies, Production and Application. Springer-Verlag, Berlin 2001. Für die Zwecke der vorliegenden Erfindung umfasst der Begriff "IgY-Antikörper" sowohl polyklonale als auch monoklonale IgY-Antikörper, die beispielsweise durch Immunisierung von eierlegendem Geflügel und anschließender Isolierung aus dem Eidotter hergestellt werden können oder durch biotechnologische Verfahren wie Klonierung, Expression und Aufreinigung. Bevorzugt handelt es sich um polyklonale IgY-Antikörper.

Unter Fragmenten von IgY-Antikörpern werden Teilbereiche intakter IgY-Antikörper verstanden, die die Antigenbindungsstellen des zugrunde liegenden Antikörpers umfassen und im Wesentlichen dieselbe Bindungsspezifität aufweisen wie der entsprechende intakte IgY-Antikörper. Solche Fragmente können beispielsweise enzymatisch erzeugt werden, z.B. durch Spaltung intakter IgY-Antikörper mittels geeigneter Proteasen wie Thrypsin. Fragmente von IgY-Antikörpern können aber auch durch biotechnologische und/oder mechanische Verfahren hergestellt werden. Eine besondere Form der Fragmente stellen die Fab-Fragmente dar, bei denen der Fc-Teil des entsprechenden intakten IgY-Antikörpers entfernt worden ist. Dabei umfasst der Begriff "Fab-Fragment" sowohl Fragmente mit einer Fab-Region als auch Fragmente mit zwei Fab-Regionen (sog. F(ab)₂-Fragmente).

Bevorzugt enthält die erfindungsgemäße Zusammensetzung polyklonale IgY-Antikörper, Fragmente und/oder Fab-Fragmente davon, die spezifisch an ein Peptid mit der Aminosäuresequenz mit der SEQ ID No. 1 binden. Der Vorteil polyklonaler IgY-Antikörper besteht darin, dass diese in der Regel mehr als ein Epitop auf dem Peptid erkennen und damit besonders effektiv die Bindung von tTG2 modifiziertem Gliadin an HLA-Proteine inhibieren.

Die erfindungsgemäße IgY-Zusammensetzung zeichnet sich dadurch aus, dass diese IgY-Antikörper, Fragmente und/oder Fab-Fragmente davon enthält, die spezifisch an ein Peptid mit einer Aminosäuresequenz mit der SEQ ID No. 1 binden. Die erfindungsgemäße IgY-Zusammensetzung zeichnet sich dadurch aus, dass die IgY-Zusammensetzung IgY-Antikörper, Fragmente und/oder Fab-Fragmente davon aufweist, die spezifisch an ein Peptid binden können, welches aus einer Aminosäuresequenz mit der SEQ ID No. 1 besteht.

Bevorzugt enthält die erfindungsgemäße IgY-Zusammensetzung IgY-Antikörper, Fragmente und/oder Fab-Fragmente davon, die spezifisch an ein Peptid mit der Aminosäuresequenz mit der SEQ ID No. 1 binden mit einer K*_{D}* von ≤ 500nM.

Als besonders vorteilhaft hat es sich erwiesen, wenn die erfindungsgemäße IgY-Zusammensetzung neben IgY-Antikörpern, Fragmenten und/oder Fab-Fragmenten davon, die spezifisch an ein Peptid mit der Aminosäuresequenz mit der SEQ ID No. 1 binden, noch weitere IgY-Antikörper, Fragmente und/oder Fab-Fragmente davon aufweist, die spezifisch an humane tTG2 mit der SEQ ID No. 5 binden.

Die erfindungsgemäße IgY-Zusammensetzung lässt sich grundsätzlich nach bekannten Verfahren für die Herstellung von spezifischen IgY-Antikörpern bzw. IgY-Präparationen oder -Zusammensetzungen herstellen, siehe z.B. Rüdiger Schade, Irene Behn, Michael Erhard: Chicken Egg YolkAntibodies, Production and Application. Springer-Verlag, Berlin 2001.

Das Verfahren kann dabei folgende Schritte umfassen:
- Bereitstellung des für die Immunisierung zu verwendenden Antigens, also beispielsweise eines Peptids mit der Aminosäuresequenz 1 und/oder 6:
- Immunisierung eierlegenden Geflügels mit dem Antigen;
- Sammeln der Eier des immunisierten Geflügels;
- Herstellen der IgY-Zusammensetzung, wobei das Eigelb der gesammelten Eier aufgearbeitet wird.

Im erfindungsgemäßen Verfahren kann für die Immunisierung des eierlegenden Geflügels als Antigen ein Peptid verwendet werden, welches eine Aminosäuresequenz mit der SEQ ID No. 1 enthält oder daraus besteht. Das Peptid für die Immunisierung kann neben der Aminosäuresequenz mit der SEQ ID No. 1 ggf. noch weitere Aminosäuresequenzen aufweisen, wie z.B. in den Aminosäuresequenzen mit der SEQ ID No. 2, 3 und/oder 4. Das Peptid kann beispielsweise auch mehrere Kopien einer Sequenz mit der SEQ ID No. 1, 2, 3 und/oder 4 umfassen.

Eine IgY-Zusammensetzung, die sowohl IgY-Antikörper aufweist, die spezifisch sind für ein Peptid mit der SEQ ID No. 1 als auch IgY-Antikörper, die spezifisch humane tTG2 binden können, lässt sich beispielsweise dadurch herstellen, dass:
- IgY-Antikörperfraktionen aus getrennten Immunisierungen gemischt werden, wobei in einer ersten Immunisierung ein Peptid enthaltend oder bestehend aus einer Aminosäuresequenz mit der SEQ ID No. 1 eingesetzt wurde und in einer zweiten Immunisierung ein Antigen umfassend mindestens ein Fragment der humanen tTG2 verwendet wurde;
- für die Immunisierung eine Mischung umfassend ein Peptid mit der SEQ ID No. 1 und ein Antigen umfassend mindestens ein Fragment der humanen tTG2 verwendet wurde; oder
- für die Immunisierung ein Peptid verwendet wird, welches sowohl die Aminosäuresequenz mit der SEQ ID No. 1 als auch mindestens ein Fragment der humanen tTG2 aufweist, z.B. als Fusionsprotein.

Dazu kann für die Immunisierung und/oder Herstellung der erfindungsgemäßen IgY-Zusammensetzung ein Peptid verwendet werden, welches zusätzlich zur Aminosäuresequenz mit der SEQ ID No. 1 eine Sequenz von mindestens 25 aufeinanderfolgenden Aminosäuren, bevorzugt von mindestens 100 aufeinanderfolgenden Aminosäuren, aus der Sequenz des humanen tTG2 mit der SEQ ID No. 5 aufweist. Dabei können die Teilbereiche, die Bestandteile des Peptids sind, grundsätzlich aus jedem Teil der tTG2-Sequenz mit der SEQ ID No. 5 ausgewählt sein. Bevorzugt umfasst das Peptid neben einer oder mehrerer Aminosäuresequenzen mit der SEQ ID No. 1, 2, 3 und/oder 4 zusätzlich eine oder mehrere Teilsequenzen der tTG2 oder eine oder mehrere Kopien der gesamten Sequenz der tTG2 mit der SEQ ID No. 5. Zusätzlich kann das Peptid für die Immunisierung und/oder Herstellung der erfindungsgemäßen IgY-Zusammensetzung noch weitere Aminosäuresequenzen umfassen, wie beispielsweise ein His-Tag, insbesondere ein 6xHis-Tag. Insbesondere kann das Peptid eine Sequenz mit der SEQ ID No. 6 [SEQ ID Nos 5 + 2] oder mit der SEQ ID No. 7 [SEQ ID Nos 5 + 2 + 2] aufweisen oder daraus bestehen. Ein Vorteil der Immunisierung mit einem solchen Fusionsprotein besteht darin, dass in einer einzigen Immunisierung polyklonale IgY-Antikörperfraktionen gewonnen werden können, die IgY-Antikörper enthalten, die sowohl an das Peptid mit der SEQ ID No. 1 binden, als auch IgY-Antikörper, die an humane tTG2 binden und diese blockieren. Es wird so eine zweifache Wirkung erzielt. Einerseits wird pathogenes tTG2 modifiziertes Gliadin gebunden und daran gehindert an HLA-Proteine des Patienten zu binden, andererseits wird die enzymatische Funktion der tTG2 inhibiert und somit die Bildung von pathogenem tTG2 modifiziertem Gliadin unterbunden.

Im offenbaren Verfahren handelt es sich bei dem eierlegenden Geflügel bevorzugt um Hühner, Enten oder Gänse. Besonders bevorzugt werden Hühner eingesetzt. Um zu einem möglichst reinen Produkt zu gelangen, können insbesondere spezifiziert pathogenfreie Hühner (sog. SPF-Hühner) verwendet werden.

Das eierlegende Geflügel wird mit dem Antigen in an sich bekannter Art und Weise immunisiert.

Die Eier der immunisierten Tiere werden gesammelt und für die Herstellung der erfindungsgemäßen IgY-Zusammensetzung eingesetzt.

Je nach gewünschtem oder erforderlichem Reinheitsgrad, kann die Herstellung der IgY-Zusammensetzung eine Reihe von verschiedenen Schritten umfassen. Zunächst kann das die IgY-Antikörper enthaltende Eigelb vom Eiweiß getrennt werden. Das vom Eiweiß getrennte Eigelb kann bereits für einige Anwendungen als erfindungsgemäße IgY-Zusammensetzung eingesetzt werden, wie z.B. als Zusatzstoff für Nahrungs- und/oder Lebensmittel. Es ist auch möglich das gewonnene Eigelb weiter aufzuarbeiten um reinere IgY-Zusammensetzungen zu erhalten. Die IgY-Antikörper können beispielsweise Affinitätsgereinigt werden und/oder durch Größenausschlußverfahren von anderen Bestandteilen des Eigelbs befreit werden. Dem Fachmann sind Verfahren und Methoden bekannt, um spezifische IgY-Antikörper aus Eigelb zu isolieren und aufzureinigen.

Die Erfindung bezieht sich auf eine IgY-Zusammensetzung, die nach dem offenbaren Verfahren gewonnen wurde.

Die erfindungsgemäße IgY-Zusammensetzung ist dadurch gekennzeichnet sein, dass die IgY-Zusammensetzung erhalten wird durch ein erfindungsgemäßes Verfahren enthaltend die Schritte:
a) Immunisierung eierlegenden Geflügels mit einem Peptid enthaltend oder bestehend aus einer Aminosäuresequenz mit der SEQ ID No. 1;
b) Sammeln der Eier des immunisierten Geflügels; und
c) Herstellen der IgY-Zusammensetzung wobei das Eigelb der gesammelten Eier aufgearbeitet wird.

Dabei kann die Immunisierung des eierlegenden Geflügels mit einem Peptid enthaltend oder bestehend aus einer Aminosäuresequenz mit der SEQ ID No. 1, 6 oder 7 erfolgen oder mit einer Mischung von Peptiden umfassend Peptide enthaltend eine Aminosäuresequenz mit der SEQ ID No. 1 und Peptide enthaltend eine Aminosäuresequenz mit der SEQ ID No. 5. Gegenstand der vorliegenden Erfindung sind ebenfalls Lebensmittel und Nahrungsmittel sowie Lebens- und Nahrungsmittelzusätze, die die erfindungsgemäße IgY-Zusammensetzung enthalten.

Die Erfindung bezieht sich auch auf eine pharmazeutische Zusammensetzung enthaltend die erfindungsgemäße IgY-Zusammensetzung und ggf. einen oder mehrere medizinisch verträgliche Hilfsstoffe.

Die pharmazeutische Zusammensetzung der vorliegenden Erfindung kann je nach gewünschter Verabreichungsart unterschiedlich ausgebildet sein. Bevorzugt ist die erfindungsgemäße IgY-Zusammensetzung bzw. die erfindungsgemäße pharmazeutische Zusammensetzung für die orale Verabreichung formuliert. Die Formulierung für die orale Verabreichung kann Formulierungen umfassen bei denen die IgY-Zusammensetzung mit einer magensaft-resistenten Beschichtung umschlossen ist, die es erlaubt, dass die IgY-Zusammensetzung in den Darm gelangt und besser resorbiert werden kann. Eine IgY-Erkrankung, Störung oder eines Zustands oder von Symptomen, die mit einer solchen Erkrankung, Störung oder eines solchen Zustands verbunden sind, verringert wird.

Die Verwendung der erfindungsgemäßen IgY-Zusammensetzung oder pharmazeutischen Zusammensetzung zur Therapie von Zöliakie kann sich dadurch auszeichnen, dass die erfindungsgemäße Zusammensetzung durch den Patienten selbst verabreicht wird (sog. Selbstadministration oder Selbstverabreichung). Dadurch wird sichergestellt, dass der Patient nur dann einer Medikation ausgesetzt wird, wenn er diese auch wirklich benötigt. Der Patient weiß meist vorab, ob er sich einer Situation aussetzen muss, in der er damit rechnen muss Gluten-haltige oder transglutaminierte Nahrungsmittel aufzunehmen. Der Patient ist meist selbst am besten in der Lage zu bemerken, ob er kürzlich Gluten-haltige oder transglutaminierte Nahrungsmittel zu sich genommen hat. In all diesen Fällen kann der Patient selbständig mit der Verabreichung einer Dosis der erfindungsgemäßen IgY-Zusammensetzung bzw. der pharmazeutischen Zusammensetzung reagieren. Dabei ist bevorzugt jede Selbstverabreichung ausgelöst von einem erwarteten oder tatsächlichen Verzehr einer Gluten-haltigen oder transglutaminierten Zusammensetzung durch den Patienten. Um eine besonders effektive Wirkung der verabreichten Dosis zu gewährleisten erfolgt eine Selbstverabreichung bevorzugt in einem Zeitfenster von nicht mehr als 5h vor bis nicht mehr als 5h nach einem erwarteten oder tatsächlichen Verzehr eines Gluten-haltigen oder transglutaminierten Nahrungsmittels.

Die vorliegende Offenbarung bezieht sich auf ein Verfahren zur Therapie von Zöliakie, Rheuma und/oder Weizenallergie, wobei einem Patienten, der einer solchen Therapie bedarf, eine wirksame Dosis der erfindungsgemäßen IgY-Zusammensetzung oder der erfindungsgemäßen pharmazeutischen Zusammensetzung verabreicht wird.

Gemäß vorliegender Erfindung wird die erfindungsgemäße IgY -Zusammensetzung oder die erfindungsgemäße pharmazeutische Zusammensetzung vorzugsweise in einer wirksamen Dosis verabreicht. Eine "wirksame Dosis" ist die Dosis der erfindungsgemäßen Zusammensetzung, die bei Verabreichung an einen Patienten eine messbare therapeutische Wirkung im Hinblick auf die fragliche Krankheit bewirkt. Bei der vorliegenden Erfindung ist eine wirksame Dosis diejenige Dosis der erfindungsgemäßen Zusammensetzung, die bei Verabreichung an einen Patienten eine therapeutische Wirkung im Hinblick auf die Therapie von Zöliakie, Rheuma und/oder Weizenallergie zeigt. Vorzugsweise wird die Zusammensetzung in einer Dosis von nicht mehr als 5 mg/kg Körpergewicht pro Behandlung oder Verabreichung verabreicht. Insbesondere kann die erfindungsgemäße Zusammensetzung in einer Dosis von 0,1 µg/kg bis 5000 µg/kg Körpergewicht pro Behandlung oder Verabreichung, vorzugsweise 1 µg/kg bis 2000 µg/kg Körpergewicht pro Behandlung oder Verabreichung verabreicht werden. Um das Auftreten akuter Nebenwirkungen zu verhindern, wird empfohlen, die erfindungsgemäße Zusammensetzung in einer kumulativen maximalen Tagesdosis von nicht mehr als 10 mg/kg Körpergewicht einzusetzen.

Figuren:
- Fig1. zeigt: die Inhibition der Zöliakie-spezifischen IgA und IgG Serumantikörper von 15 Zöliakiepatienten durch anti-tTG-GD3-IgY und anti-CD3-IgY Hühnerantikörper.
- Fig2. zeigt: die spezifische Bindung von anti-tTG-CD3-IgY und CD3-IgY an tTg2 modifiziertes Weizengliadin.
- Fig3 zeigt: die spezifische Inhibition der Enzymaktivitäten von tTg2 durch Anti-tTG-CD3-IgY.

### Beispiele:

### Herstellung und Aufreinigung von CD3, tTG und CD3tTG

CD3 mit der SEQ ID No. 4 wurde synthetisch hergestellt und am C-terminalen Lysinrest biotinyliert.

Humanes tTG2 mit der SEQ ID No. 5, im Folgenden als tTG bezeichnet, wurde für die rekombinante Expression in einen Vektor mit spaltbarem 6xHis-tag kloniert und nach Standardprotokoll exprimiert, mittels NiNTA-Säulenaufreinigung isoliert und der 6xHis-tag wurde wieder entfernt.

CD3tTG mit der SEQ ID No. 6, im Folgenden auch als CDtTG bezeichnet, wurde für die rekombinante Expression ebenfalls in einen Vektor mit C-terminalem 6xHis-tag kloniert und nach Standardprotokoll exprimiert, mittels NiNTA-Säulenaufreinigung isoliert und der His-tag wurde wieder abgespalten.

### Seren Inhibitionsversuch

Es wurde die Inhibition der Zöliakie-spezifischen IgA und IgG Serumantikörper von 15 Zöliakiepatienten durch anti-tTG-CD3-IgY und anti-CD3-IgY Hühnerantikörper untersucht. Für die Inhibition der CD3 spezifischen Antikörper wurde ein CD3 Peptid-ELISA entwickelt, bei dem biotinyliertes CD3 mit der zunächst an eine mit neutrAvidin beschichtete Mikrotiterplatter gekoppelt wurde und dann folgendes Protokoll verwendet wurde:

### NeutrAvidin-CD3 Peptid ELISA

Zunächst wurden die Wells der Mikrotiterplatte mit PBS/5% MP Puffer über Nacht geblockt. Anschließend folgte der Auftrag der biotinylierten Peptide mit je 500 pmol/well in PBS Puffer. Nach einer Inkubation von 2 h wurden die CD3/neutrAvidinbeschichteten Platten 4 × mit PBS/0,1% Tween gewaschen und dann mit dem Patientenserum, mit einer Verdünnung von 1:800 in PBS/2% MP, 1 h lang inkubiert. Es wurde danach wiederum 4 gewaschen, gefolgt vom Auftrag des Peroxidasekonjugierten 2.AK mit einer 1:5000 Verdünnung. Die Inkubation dauerte ebenfalls 1 h. Abschließend erfolgte wiederum 4 × waschen gefolgt vom Auftrag des Substrats. Die Reaktion mit Entwicklung der Blaufärbung wurde nach 5 min mit 0,5 M Schwefelsäure abgestoppt. Die resultierende Gelbfärbung wurde mittels des ELISA Readers photometrisch bei einer Messwellerrlänge von 450 nm gegen die Referenzwellenlänge von 620 nm gemessen und mit Hilfe der Software Magellan dargestellt. Von allen Lösungen wurden pro Well 100 µl aufgetragen. Das Blocken und die einzelnen Waschschritte erfolgten mit je 300 µl pro Well. Die Inkubationen der Mikrotiterplatten mit Peptid, Patientenserum sowie des 2.AK erfolgten unter schütteln bei RT.

Für die Blockierungsversuche wurde mit 5µg IgY (entspricht ca. 0,5-1µg spezifischer CD3 AK) in 100µl PBS für 30 Min nach der CD3 Beschichtung inkubiert und danach mit 4 × mit Pos/0,1% Tween gewaschen.

### tTG-ELISA-Protokoll:

Für den Nachweis tTG2- IGY Inhibition Antikörper wurde ein spezifischer ELISA entwickelt, bei dem rekombinantes tTg2 zunächst an eine MaxiSorb Mikrotiterplatte (Nunc) gekoppelt wurde und dann wurde folgendesProtokoll verwendet: A) Kopplungspuffer: 100 mM Tris, 10 mM NaCl pH 7,8 B) Waschpuffer: 50 mM Tris-HCl, 150 mM NaCl, 10 mM EDTA, 0,1% Tween 20 pH 7,4 C) Absättigungspuffer 50 mM Tris-HCl, 150 mM NaCl, 0,5% BSA, 3% Saccharose pH 7,4 D) Serumverdünnungspuffer: 50 mM Tris-HCl, 150 mM NaCl, 0,5% Tween 20 pH 7,4

### Durchführung:

Beschichtung auf MaxiSorp-Platten von Nunc: Beschichtungsmenge: tTg2 wurden jeweils in einer Konzentration von 0,5 µg/well eingesetzt.Beschichtungsvolumen: 100 µl/well. Alle tTG's werden in Kopplungspuffer A entsprechend verdünnt. Für die Beschichtung werden Platten ü.N. bei 4°C inkubiert. Bei der ELISA-Durchführung werden Blank und 2.AK-Kontrollen mitgeführt. Die OD-Werte der Blank- und 2.AK-Kontrollen werden bei der Auswertung des ELISAs abgezogen. Die Platten werden nach der Beschichtung mit 3 × 300 µl/well Waschpuffer B gewaschen. Ein Waschschritt entspricht je 3 min 600 rpm auf dem ELISA-Schüttler. Anschließend blockt man die Platten mit 300 µl/well Absättungspuffer C für 2 h bei RT. Für die Blockierungsversuche wurde mit 5µg IgY (entspricht ca. 0,5-1 µg spezifischer CD3tTG AK) in 100µl Puffer D verdünnt für 30 Min nach der tTg Beschichtung inkubiert danach mit Puffer B gewaschen.

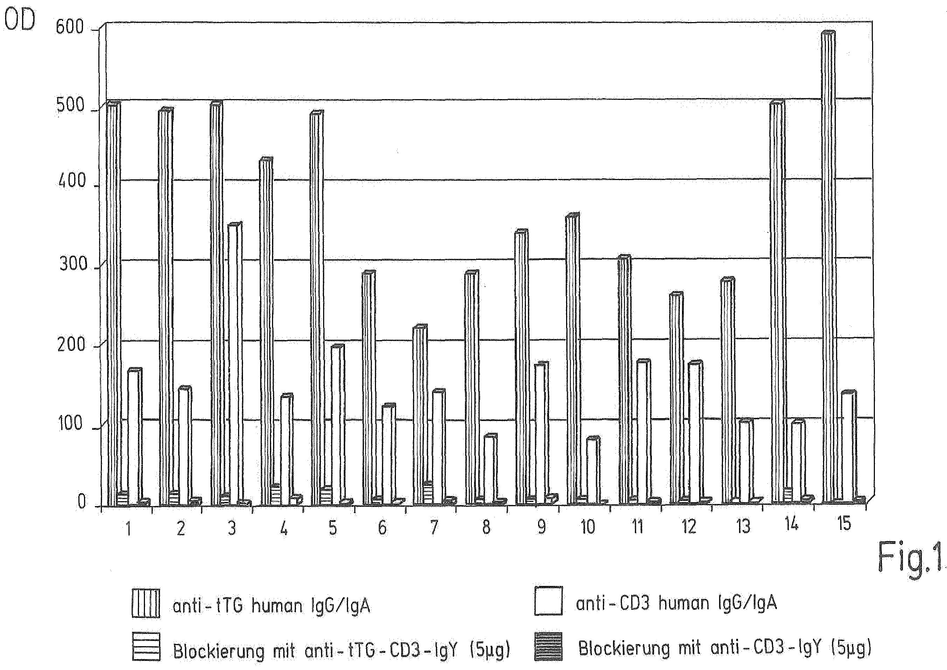

Die Seren werden 1:800 in Serumverdünnungspuffer D verdünnt und mit 100 µl/well nach dem Blocken direkt auf Platten gegeben. Inkubation für 1 h bei RT unter Schütteln 5 × mit 300 µl/lwell mit Waschpuffer B waschen. 2.AK: - <hlgA HRP von Dako wird 1:1500 in Waschpuffer B verdünnt und 100 µlwell eingesetzt; - <hlgG>HRP von Dako wird in einer Verdünnung von 1:5000 ebenfalls in Waschpuffer B und 100 µl/well verwendet. Inkubation für 1 h bei RT unter Schütteln. 4 × mit 300 µl/well mit Waschpuffer B waschen. Mit 100 µl/well TMB-Substrat (SeramunBlue fast) 5min. reagieren lassen. Anschließend mit 100 µl/well Stopplösung (0,5 M H2SO4) stoppen und im ELISA-Reader bei 450 nm auswerten. 3. Kohorte der Zöliakie-Patienten-Seren Im Zuge dieser Arbeit wurden humane Patientenseren von 15 Patienten mit positivem Zöliakie Befund, verschiedenen Alters, Geschlecht und pathologischer Charakteristik eingesetzt. Alle verwendeten Seren konnten in ihrem anti-tTG2 und -CD3 Signal unter Cut off inhibiert werden, siehe Figur 1.

### IgY-Aufreinigung

Für eine verbesserte und schnellere Aufreinigung der IgY-Antikörper der rekombinanten Fusionsproteine aus Hühnereiern wurde das optimierte Chloroform- Polyethylene glycol Protokoll nach Gamenisch et al. verwendet. Das Eigelb wird im ersten Schritt vom Eiweiß getrennt. Danach wird das flüssige innere Eigelb auf sterilem Filterpapier, durch Perforation des Dottersackes mittels Pippette getrennt und in ein 50ml Falkontube überführt. Das 50 ml Falkontube mit vorgelegtem Eigelb wird danach auf 25ml mit PBS-Puffer aufgefüllt und gevortext. Anschließend wird das Eigelb-PBS-Gemisch mit 20 ml Chloroform versetzt, gevortext und geschüttelt bis ein homogenes Gemisch entsteht. Nach dem Zentrifugieren bei 1200 xg für 30 min, wird der Überstand in ein neues Zentrifugen Röhrchen überführt und mit Polyethylene glycol 6000 (Fa. Fluka) bis auf eine Endkonzentration von 12% (w/v) gebracht. Nach der Zentrifugation bei 15700 xg für 10 min, wird das Pellet mit PBS resuspendiert und ein Teil auf Reaktivität im Westernblott und ELISA mit dem entsprechenden Protein oder Peptid kontrolliert, der Rest im Kryoröhrchen bei -80°C gelagert.

### Herstellung von polyklonalem IGY Antikörpern gegen CD3tTG und CD3

Es wurden Hühner für die Herstellung von polyklonalen Antikörpern ausgewählt, weil hier große Mengen IgY-Antikörper aus dem Eigelb (bzw. Eidotter Y von Yolk) isoliert werden können und für die Immunisierung nur kleinere Antigenmengen notwendig. Pro Immunisierungsschritt wurden 100 -bis 150µg Antigen (CD3 Peptide gekoppelt an KLH und Rekombinate tTG-CD3), welche in 250µl Elutionspuffer vorlagen, eingesetzt. Diese Portion wurde zu gleichen Teilen mit Freundschen Adjuvans versetzt Die Hühner wurden durch intramuskulär Injektion der über 2-mal HisPur™ Cobalt-Resin und 1-mal Q Sepharose® Fast Flow gereinigten rekombinanten Fusionsproteine zur Bildung von Antikörpern angeregt. Boosterimpfungen erfolgten nach 14 und 38 Tagen. Nach insgesamt 64 Tagen wurden die Hühnereier gewonnen.

Das kommerziell erworbene Weizengliadin (Sigma) wurde mit an die Wells gekoppelt (2ug). Dazu wurde das Gliadin zuvor in 8 M Harnstoff gelöst (60 µg/ml) und entsprechende Verdünnungen hergestellt. Es wurden nach dem Koppeln der Proteine diese durch das Enzym Transglutaminase modifiziert. Die Transglutaminase katalysiert die Deaminierung von Glutamin. Dies führt zur Umwandlung von Glutamin zu Glutamat. Für die Modifizierung wurde die ELISA Platte mit PBS-Puffer gewaschen. Nach Zugabe von 100ul Modifikations-Puffer wurden 20 µl tTG2 (250 µg/ml) dazu pipettiert und bei 37 °C je 120 µl/well eingesetzt und für 2 h inkubiert. Danach wurde mit 2x1 min mit PBS gewaschen

### Modifikations-Puffer

10 mM DTT
10 mM CaCl₂
in TBS

Die Blockierung erfolgte anschließend für 1 h mit je 150 µl 5 % Milchpulver/PBS. Danach wurde mit 2x1 min mit PBS gewaschen. Nun wurden je 120 µl Anti-CD3 und anti-CD3-tTG IgY Antikörper in 5 % Milchpulver 1:500 und 1:1000 verdünnt in die Wells pipettiert und für 1 h bei RT inkubiert. Nach einem Waschschritt von 4x1 min mit PBS, folgte die Zugabe von je 120 µl HRP-gekoppelten Sekundärantikörper (Anti-Huhn-AK 1:2000 für Anti-CD3 und CD3tTG) in 5 % Milchpulver. Die Inkubation erfolgte für 30 min. Im Anschluss wurde 5x1 min mit PBS gewaschen. Die Visualisierung der Immunreaktion erfolgte durch Zugabe von je 100 µl TMB für 5 min und anschließendem Abstoppen der Reaktion mit je 100 µl 0,5 M H₂SO₄. Gemessen wurde bei 450 nm gegen eine Referenzwellenlänge von 620 nm am ELISA-Plattenphotometer. Bei allen durchgeführten ELlSA-Tests erfolgten die Inkubationen und Waschschritte auf einem Schüttler (Titramax 1400, Heidolph) bei 600 rpm. Es wurde stets eine Doppelbestimmung vorgenommen und folgende Kontrollen immer mitgeführt.

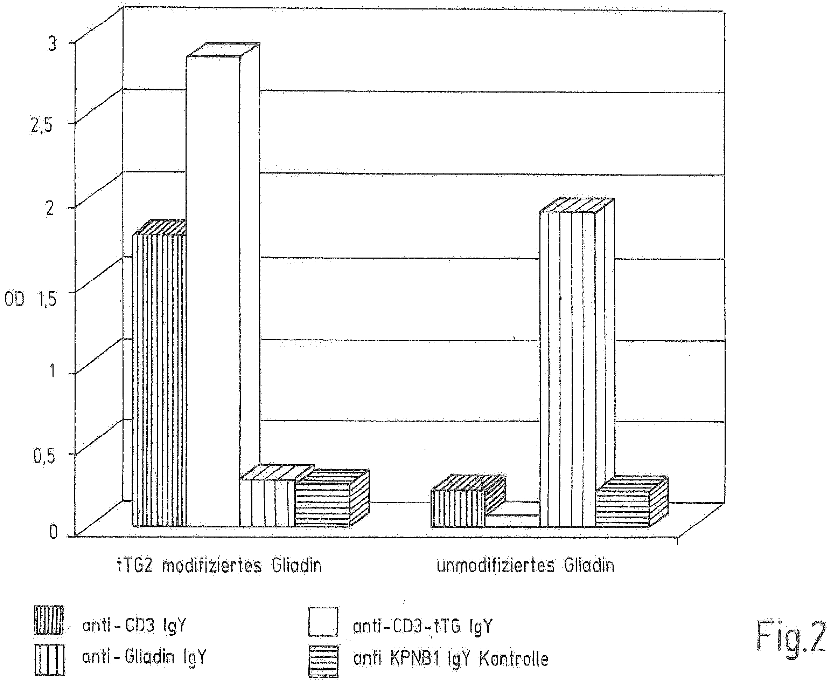

Die Ergebnisse sind in Figur 2 dargestellt. Anti-CD3 IgY und anti-CD3-tTG IgY Antikörper erkennen spezifisch die tTG-modifizierte Form des Weizengliadins, während die anti-CD3 IgY und anti-CD3-tTG IgY Antikörper keine spezifische Reaktivität mit nicht pathogenem, unmodifiziertem Gliadin zeigen. Ein IgY Antikörper der gegen unmodifiziertes Gliadin gerichtet ist, zeigt eine spezifische Bindung an unmodifiziertes Gliadin, während tTG2 modifiziertes Gliadin von diesem Antikörper nicht erkannt wird.

### tTG2-Enzym Aktivitätstest

Die enzymatische Aktivität der tTG in An- und Abwesenheit von anti-CD3-tTG IgY Antikörpern wurden durch Messung des Einbaus von Casein quantifiziert. Es wurde Casein (17 mM) mit 30 mM monodansyl Cadaverin in einem Gesamtvolumen von 100 ml 0,1 M Tris-HCl, 0,15 M NaCl, 5 mM CaCl2, pH 7,5 inkubiert. Der Einbau von monodansyl Cadaverin (N-(5-aminopentyl)-5-dimethylamino-1-naphthalinsulfonamide von Sigma in Rinder-a-Casein führt zu einer erhöhte Intensität der Fluoreszenz der Dartsyl-Gruppe. Für Inhibitionsstudien der anti-CD3-tTG IgY Antikörper wurden in unterschiedlichen Konzentrationen im Bereich von 1 und 2,5 mg aufgenommen. Die Reaktion wurde durch Zugabe von 0,5 mg humaner rekombinanter tTG bei 37 ° C gestartet. Die Anregung erfolgte bei 360 nm und die Zunahme der Fluoreszenz bei 550 nm mit einem Fluoreszenz Spektralphotometer gemessen. tTG-Aktivität wurde als Prozentsatz der verbleibenden Aktivität in Korrelation zu den Experimenten mit Zugabe von Kontroll-Antikörper berechnet.

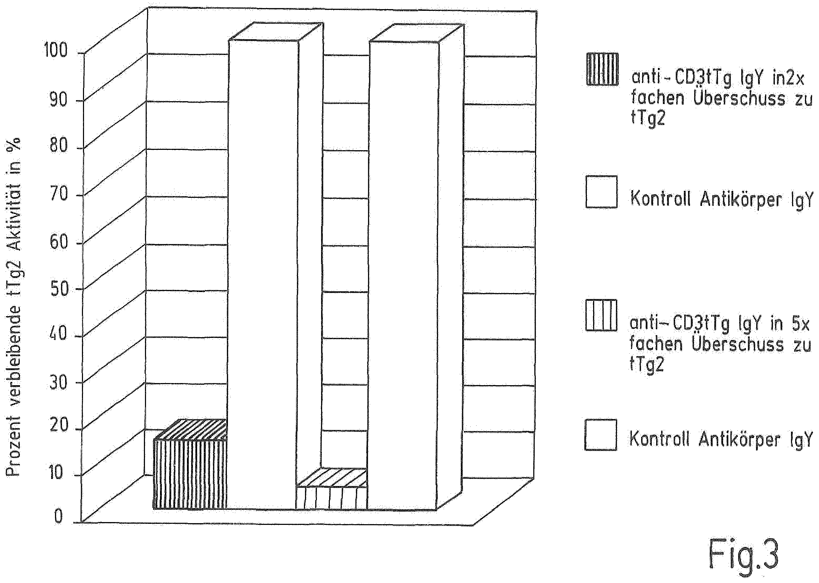

Wie in Figur 3 gezeigt, ist anti-CD3-tTG IgY Antikörper in der Lage die Enzymaktivität der tTG2 effektiv zu blockieren während ein irrelevanter IgY-Kontrollantikörper keinen Einfluss auf die tTG2-Aktivität zeigt.

Anti-CD3-tTG IgY zeigten eine Inhibition der tTG2 Aktivität von > 95 %. Durch diesen inhibitorischen Effekt kann anti-CD3-tTG IgY die pathologische T-Zell Aktivierung und Vermehrung blockieren.

## Patentansprüche

1. IgY-Zusammensetzung, wobei die IgY-Zusammensetzung IgY-Antikörper, Fragmente und/oder Fab-Fragmente davon enthält, die spezifisch an ein Peptid mit einer Aminosäuresequenz mit der SEQ ID No. 1 binden, **dadurch gekennzeichnet, dass** die IgY-Zusammensetzung erhalten wird durch ein Verfahren enthaltend die Schritte:
a) Immunisierung eierlegenden Geflügels mit einem Peptid enthaltend oder bestehend aus einer Aminosäuresequenz mit der SEQ ID No. 1;
b) Sammeln der Eier des immunisierten Geflügels; und
c) Herstellen der IgY-Zusammensetzung wobei das Eigelb der gesammelten Eier aufgearbeitet wird.

2. IgY-Zusammensetzung nach Anspruch 1, **dadurch gekennzeichnet, dass** die Immunisierung des eierlegenden Geflügels mit einem Peptid enthaltend oder bestehend aus einer Aminosäuresequenz mit der SEQ ID No. 1, 6 oder 7 erfolgt oder mit einer Mischung von Peptiden umfassend Peptide enthaltend eine Aminosäuresequenz mit der SEQ ID No. 1 und Peptide enthaltend eine Aminosäuresequenz mit der SEQ ID No. 5.

3. IgY-Zusammensetzung nach einem der Ansprüche 1 oder 2, **dadurch gekennzeichnet, dass** die Aufarbeitung des Eigelbs eine Isolierung von IgY-Antikörpern aus dem Eigelb umfasst.

4. IgY-Zusammensetzung nach einem der Ansprüche 1, 2 oder 3, **dadurch gekennzeichnet, dass** es sich bei dem Geflügel um Hühner handelt, bevorzugt um SPF-Hühner.

5. Lebensmittel oder Lebensmittelzusatz enthaltend eine IgY-Zusammensetzung nach einem der Ansprüche 1 bis 4.

6. Pharmazeutische Zusammensetzung enthaltend eine IgY-Zusammensetzung nach einem der Ansprüche 1 bis 4 und mindestens einen medizinisch verträglichen Hilfsstoff.

7. Kit umfassend einen Behälter enthaltend eine IgY-Zusammensetzung nach einem der Ansprüche 1 bis 4 oder eine pharmazeutische Zusammensetzung nach Anspruch 6 sowie eine Gebrauchsanweisung.

8. IgY-Zusammensetzung nach einem der Ansprüche 1 bis 4 oder pharmazeutische Zusammensetzung nach Anspruch 6 für die Verwendung in der Therapie von Zöliakie, Rheuma und/oder Weizenallergie.

9. IgY-Zusammensetzung oder pharmazeutische Zusammensetzung für die Verwendung nach Anspruch 8,
**dadurch gekennzeichnet, dass** die IgY-Zusammensetzung nach einem der Ansprüche 1 bis 4 oder die pharmazeutische Zusammensetzung nach Anspruch 6 durch den Patienten selbst verabreicht wird.

10. IgY-Zusammensetzung oder pharmazeutische Zusammensetzung r für die Verwendung nach Anspruch 9,
**dadurch gekennzeichnet, dass** jede Selbstverabreichung ausgelöst wird von einem erwarteten oder tatsächlichen Verzehr eines Gluten-haltigen oder transglutaminierten Nahrungsmittels.

11. IgY-Zusammensetzung oder pharmazeutische Zusammensetzung für die Verwendung nach Anspruch 10,
**dadurch gekennzeichnet, dass** jede Selbstverabreichung in einem Zeitfenster erfolgt von nicht mehr als 5h vor bis nicht mehr als 5h nach einem erwarteten oder tatsächlichen Verzehr eines Gluten-haltigen oder transglutaminierten Nahrungsmittels.

12. IgY-Zusammensetzungnach Anspruch 1, **dadurch gekennzeichnet, dass** die IgY-Zusammensetzung erhalten wird durch ein Verfahren enthaltend die Schritte:
a) Immunisierung eierlegenden Geflügels mit einem Peptid enthaltend oder bestehend aus einer Aminosäuresequenz mit der SEQ ID No. 2, 3 und/oder 4;
b) Sammeln der Eier des immunisierten Geflügels; und
c) Herstellen der IgY-Zusammensetzung wobei das Eigelb der gesammelten Eier aufgearbeitet wird.

## Claims

1. An IgY composition, wherein the IgY composition contains IgY antibodies, fragments and/or Fab fragments thereof that specifically bind to a peptide having an amino acid sequence of SEQ ID No. 1, **characterized in that** said IgY composition is obtained by means of a method comprising the following steps:
a) immunizing egg-laying poultry with a peptide containing or consisting of an amino acid sequence of SEQ ID No. 1;
b) collecting the eggs of the immunized poultry; and
c) preparing the IgY composition, wherein the egg yolk of the collected eggs is processed.

2. The IgY composition according to Claim 1, **characterized in that** the immunization of the egg-laying poultry is performed with a peptide containing or consisting of an amino acid sequence of SEQ ID Nos. 1, 6, or 7 or with a mixture of peptides comprising peptides containing an amino acid sequence of SEQ ID No. 1 and peptides containing an amino acid sequence of SEQ ID No. 5.

3. The IgY composition according to any one of Claims 1 or 2, **characterized in that** the step of processing the egg yolk includes isolating IgY antibodies from the egg yolk.

4. The IgY composition according to any one of Claims 1, 2 or 3, **characterized in that** the poultry is chicken, preferably SPF chicken.

5. A food or food additive containing an IgY composition according to any one of Claims 1 to 4.

6. A pharmaceutical composition containing an IgY composition according to any one of Claims 1 to 4 and at least one medically compatible adjuvant.

7. A kit comprising a container containing an IgY composition according to any one of Claims 1 to 4 or a pharmaceutical composition according to Claim 6 and instructions for use.

8. The IgY composition according to any one of Claims 1 to 4 or the pharmaceutical composition according to Claim 6 for use in the therapy of celiac disease, rheumatism and/or wheat allergy.

9. The IgY composition or pharmaceutical composition for
use according to Claim 8,
**characterized in that** the IgY composition according to any one of Claims 1 to 4 or the pharmaceutical composition according to Claim 6 is self-administered by the patient.

10. The IgY composition or pharmaceutical composition for
use according to Claim 9,
**characterized in that** each self-administration is triggered by an expected or
actual consumption of a gluten-containing or transglutaminated food.

11. The IgY composition or pharmaceutical composition for
use according to Claim 10,
**characterized in that** each self-administration takes place in a time window of no more than 5 hours before and no more than 5 hours after an expected or actual consumption of a gluten-containing or transglutaminated food.

12. The IgY composition according to Claim 1, **characterized in that** said IgY composition is obtained by means of a method comprising the following steps:
a) immunizing egg-laying poultry with a peptide containing or consisting of an amino acid sequence of SEQ ID Nos. 2, 3 and/or 4;
b) collecting the eggs of the immunized poultry; and
c) preparing the IgY composition, wherein the egg yolk of the collected eggs is processed.

## Revendications

1. Composition d'IgY, la composition d'IgY comprenant des anticorps IgY, des fragments et/ou des fragments Fab de ceux-ci, lesquels se lient spécifiquement à un peptide avec une séquence d'acides aminés ayant le SEQ ID No. 1, **caractérisée en ce que** la composition d'IgY est obtenue par un procédé comprenant les étapes de :
a) immunisation d'une volaille pondeuse avec un peptide comprenant ou se composant d'une séquence d'acides aminés ayant le SEQ ID No. 1 ;
b) collecte des oeufs de la volaille immunisée ; et
c) réalisation de la composition d'IgY, le jaune d'oeuf des oeufs collectés étant traité.

2. Composition d'IgY selon la revendication 1, **caractérisée en ce que** l'immunisation de la volaille pondeuse avec un peptide comprenant ou se composant d'une séquence d'acides aminés ayant le SEQ ID No. 1, 6 ou 7 est effectuée avec un mélange de peptides comprenant des peptides comprenant une séquence d'acides aminés ayant le SEQ ID No. 1 et des peptides comprenant une séquence d'acides aminés ayant le SEQ ID No. 5 .

3. Composition d'IgY selon l'une des revendications 1 ou 2, **caractérisée en ce que** le traitement du jaune d'oeuf comprend un isolement d'anticorps d'IgY du jaune d'oeuf.

4. Composition d'IgY selon l'une des revendications 1, 2 ou 3, **caractérisée en ce que** les volailles sont, de préférence, des poules SPF.

5. Aliment ou additif alimentaire comprenant une composition d'IgY selon l'une des revendications 1 à 4.

6. Composition pharmaceutique comprenant une composition d'IgY selon l'une des revendications 1 à 4 et au moins un agent auxiliaire médicalement acceptable.

7. Ensemble comprenant un récipient comprenant une composition d'IgY selon l'une des revendications 1 à 4 ou une composition pharmaceutique selon la revendication 6, ainsi qu'un mode d'emploi.

8. Composition d'IgY selon l'une des revendications 1 à 4 ou composition pharmaceutique selon la revendication 6 pour l'utilisation dans le traitement de la maladie coeliaque, du rhumatisme et/ou de l'allergie au blé.

9. Composition d'IgY ou composition pharmaceutique pour l'utilisation selon la revendication 8,
**caractérisée en ce que** la composition d'IgY selon l'une des revendications 1 à 4 ou la composition pharmaceutique selon la revendication 6 est administrée par le patient lui-même.

10. Composition d'IgY ou composition pharmaceutique pour l'utilisation selon la revendication 9,
**caractérisée en ce que** chaque auto-administration est déclenchée par une consommation attendue ou effective d'un aliment contenant du gluten ou
transglutaminé.

11. Composition d'IgY ou composition pharmaceutique pour l'utilisation selon la revendication 10,
**caractérisée en ce que** chaque auto-administration est effectuée au cours d'une période de pas plus de 5 h avant à pas plus de 5 h après une consommation attendue ou effective d'un aliment contenant du gluten ou transglutaminé.

12. Composition d'IgY selon la revendication 1, **caractérisée en ce que** la composition d'IgY est obtenue par un procédé comprenant les étapes de :
a) immunisation d'une volaille pondeuse avec un peptide comprenant ou se composant d'une séquence d'acides aminés ayant le SEQ ID No. 2, 3 et/ou 4 ;
b) collecte des oeufs de la volaille immunisée ; et
c) réalisation de la composition d'IgY, le jaune d'oeuf des oeufs collectés étant traité.
